# EUROPÄISCHE PATENTSCHRIFT

(11) **EP 0 845 029 B1**
(45) Veröffentlichungstag und Bekanntmachung des Hinweises auf die Patenterteilung: **29.05.2002**
(21) Anmeldenummer: 96931743.7
(22) Anmeldetag: 03.08.1996
(51) Int. Cl.: C12M 1/24, C12M 3/06

(54) **VORRICHTUNG ZUR SERIEN-KULTIVIERUNG VON MIKROORGANISMEN BZW. ZELLEN IN BEGASTEN FLÜSSIGKEITSSÄULEN**
DEVICE FOR SERIAL CULTIVATION OF MICRO-ORGANISMS OR CELLS IN GASIFIED LIQUID COLUMNS
DISPOSITIF POUR LA CULTURE EN SERIE DE MICRO-ORGANISMES OU DE CELLULES DANS DES COLONNES DE LIQUIDE GAZEIFIE

(30) Priorität: 08.08.1995 DE 19529099
(43) Veröffentlichungstag der Anmeldung: 03.06.1998
(73) Patentinhaber: FORSCHUNGSZENTRUM JÜLICH GMBH, 52425 Jülich (DE)
(72) Erfinder: WEUSTER-BOTZ, Dirk, D-52070 Aachen (DE); ALTENBACH-REHM, Jutta, D-52078 Aachen (DE)
(86) Internationale Anmeldenummer: DE9601485
(87) Internationale Veröffentlichungsnummer: WO9706239

(56) Entgegenhaltungen:
- EP-A- 0 307 048

## Beschreibung

Die Erfindung bezieht sich auf eine Vorrichtung zur Serien-Kultivierung von Mikroorganismen bzw. Zellen in begasten Flüssigkeitssäulen.

Zur Durchführung von Reihenuntersuchungen auf festen Nährböden ist die Verwendung von Agarplatten gebräuchlich. Hierbei werden die Mikroorganismen als Einzelkeime auf den festen Nährboden gebracht und bei der jeweiligen Reaktionstemperatur inkubiert. Der Stofftransport (Substrat aus dem festen Nährboden und Sauerstoff aus der Gasphase) erfolgt rein diffusiv.

Das klassische Instrument zur Kultivierung von Mikroorganismen für Serienuntersuchungen stellt die Schüttelkolbentechnik dar. Hierbei lassen sich bei Reaktionsvolumina von wenigen ml bis zu 1000 ml mit Hilfe von Schüttelinkubatoren viele Experimente parallel durchfuhren. Der Energieeintrag zur Dispergierung der Flüssigphase (Medium und Mikroorgansimen) erfolgt dabei über eine kreisförmige Bewegung aller Reaktionsgefäße, die auf einem Tablett angeordnet sind. Der Sauerstoffeintrag ist hierbei nur über eine Oberflächenbegasung möglich. Der Stofftransport erfolgt in der Flüssigphase konvektiv.

Zur Miniaturisierung dierser Technik (um noch mehr Versuche parallel durchführen zu können) wurde versucht, Mikrotiterplatten als Reaktionsgefäße zu nutzen. Insbesondere Probleme bei der Verdunstung aufgrund des ungünstigeren Oberfläche / Volumenverhältnisses verhinderten eine Anwendung dieser Technik.

Eine apparatetechnisch sehr aufwendige Technik zur Durchführung von Serienuntersuchungen mit Mikroorganismen ist der parallele Einsatz von Laborfermentern. Hier werden Geräte angeboten, die eine Paralleldurchführung von bis zu 6 Fermentationen mit einem Gerät ermöglichen. Der Energieeintrag erfolgt hierbei über Standardrührorgane; über eine Begasungseinrichtung ist eine Volumenbegasung möglich.

Eine weitere Vorrichtung zur Serienkultivierung von Zellen ist aus EP-A-0 307 048 bekannt. Die in dieser Druckschrift offenbarte Vorrichtung besteht aus Kulturflaschen, die oberhalb ihres Bodens mit einer gasdurchlässigen Membran versehen sind und die über ihren Boden mit einer Gaszuführung, die Aufnahmeplätze für die Kulturflaschen aufweist, verbunden sind. Ein solcher apparativer Aufbau gewährleistet zwar durch die Sterilgaszuleitung eine ausreichende Begasung bzw. Sauerstoffversorgung der in den Gefäßen zu kultivierenden Zellen. Damit jedoch zusätzlich auch ein steriler Kultivierungsabtauf gewährieistet ist, muß die gesamte Anordnung vor der Beimpfung mit Zellen einer Sterilisation unterworfen werden. Wird zudem für eine Serienkultivierung die Anordnung mehrmals nebeneinander und ggf. zusätzlich noch in Reihe geschaltet, wird der Sterilisationsvorgang sehr umständlich.

Es ist daher Aufgabe der Erfindung, eine relativ einfache apparative Anordnung zur Serienkultivierung zu schaffen, die eine einfache Handhabung, insbesondere für den Sterilisationsvorgang, gewährleistet.

Diese Aufgabe wird durch eine Vorrichtung gelöst, die aus Kulturflaschen besteht, die oberhalb ihres Bodens bzw. ihrer Bodenöffnung mit einer gasdurchlässigen porösen Filterplatte ausgestattet sind, deren Porenfeinheit und Hydrophobizität ausreichen, den Flüssigkeitsablauf aus einer darüber befindlichen Kulturflüssigkeitssäule zu unterbinden, und die über ihren Boden bzw. ihrer Bodenöffnung mit einer Sterilgaszuleitung, die Aufnahmeplätze für die Kulturflaschen aufweist, gasdicht verbunden bzw. zu verbinden sind. Der Aufnahmeplatzboden ist zusätzlich mit ggf. auswechselbare und/oder als Schließventil ausgebildete Anstechvorrichtungen und die Kulturflaschen mit perforierbarem Membranboden ausgestattet. Damit ist eine getrennte Sterilisation und nachträgliche Sterilverbindung von Gasverteiler und Kulturflaschen und somit eine einfache Handhabung der Anordnung insbesondere während des Sterilisationsvorganges gewährleistet.

Eine solche Anordnung einer Serie von Kulturflaschen, die über ihren Boden mit einem Gasverteiler in Verbindung stehen, kann mit unterschiedlichen Gasverteilern in Form von Schlauchzuleizungen oder Zuleitungen über Bohrungen versehen und mit einem Serien-Stativ ausgerüstet sein.

Besonders zweckmäßig ist jedoch ein als Gasverteiler wirkender, mit Sterileinlaß für Gas versehener, gasdichter Doppelboden für die Sterilgaszuleitung, dessen obere Platte mit einer Serie von Gasauslaßöffnungen aufweisenden Aufnahmeplätzen für den abgedichteten Einsatz bzw. Aufsatz der Kulturflaschen versehen ist. D.h., zwischen dem oberen Boden des Gasverteilers und dem unteren Ende der Kulturflasche, wird für eine dichte Verbindung koaxial zur Gasverbindungsbohrung des Gasverteilers gesorgt.

Weitere Besonderheiten der Erfindung ergeben sich aus den Patentansprüchen.

Gemäß der Erfindung werden insbesondere noch folgende weitere Verbesserungen erzielt:
1. Wesentliches Problem bei der Kultivierung von aeroben Mikroorganismen ist die Sauerstoffversorgung. Durch die Verwendung von begasten Säulen zur Kultivierung von Mikroorgarnismen für Reihenuntersuchungen ist aufgrund der Volumenbegasung eine weitaus bessere Sauerstoffversorgung der Mikroorganismen als im herkömmlichen Schüttelkolben möglich. Der Unterschied im Sauerstoffeintragsvermögen zwischen Oberflächenbegasung und Volumenbegasung kann je nach Oberfläche/Volumenverhältnis mehr als 1 Zehnerpotenz betragen.
2 . Das wesentliche Problem bei der Nutzung von Daten, die in kleinen Reaktionsgefäßen gewonnen werden, ist die Übertragung dieser Ergebnisse in den Reaktor. Durch die Verbesserung der Sauerstoffversorgung in der begasten Säule auf Werte, wie sie in technischen Reaktoren erhalten werden, ist eine Maßstabsüberstragung besser möglich, insbesondere, da das verwendete Verfahren zur Sauerstoffversorgung (Volumenbegasung) identisch ist. Damit kann die Entwicklungszeit von Verfahren zur Herstellung von Produkten mit Mikroorganismen verkürzt werden.
3. Gegenüber der Verwendung von parallelen Laborreaktoren zur Kultivierung von Mikroorganismen für Reihenuntersuchungen ist der apparative und damit finanzielle Aufwand bei der Verwendung von begasten Säulen bedeutend geringer.

Nachfolgend sind zur Veranschaulichung der Erfindung in ihren Besonderheiten Zeichnungen beigefügt. Es zeigen schematisch:
- Fig. 1: einen Vertikalschnitt durch eine Serienkultur-Anordnung;
- Fig. 2: eine einzelne Kulturflasche im Detail in Schnittdarstellung;
- Fig. 3: eine erfindüngsgemäße Aufnahmeplatz- und Flaschenbodenausgestaltung;
- Fig. 4: eine Unterteilung der Anordnung in unterschiedliche Begasungsreihen in Aufsicht;
- Fig. 5: einen Vergleich der parallelen Kultivierung von Corynebacterium glutamicum Bakterien zur L-Isoleucin-Herstellung in den verwendeten Kulturflaschen (begasten Säulen) und in Schüttelkolben;
- Fig. 6: einen Vergleich der volumetrischen Stoffübergangskoeffizienten (k_{L}a) für begaste Säulen, Schüttelkolben und 2 l Rührreaktor, die nach der Sulfitmethode bei 30°C bestimmt worden sind.

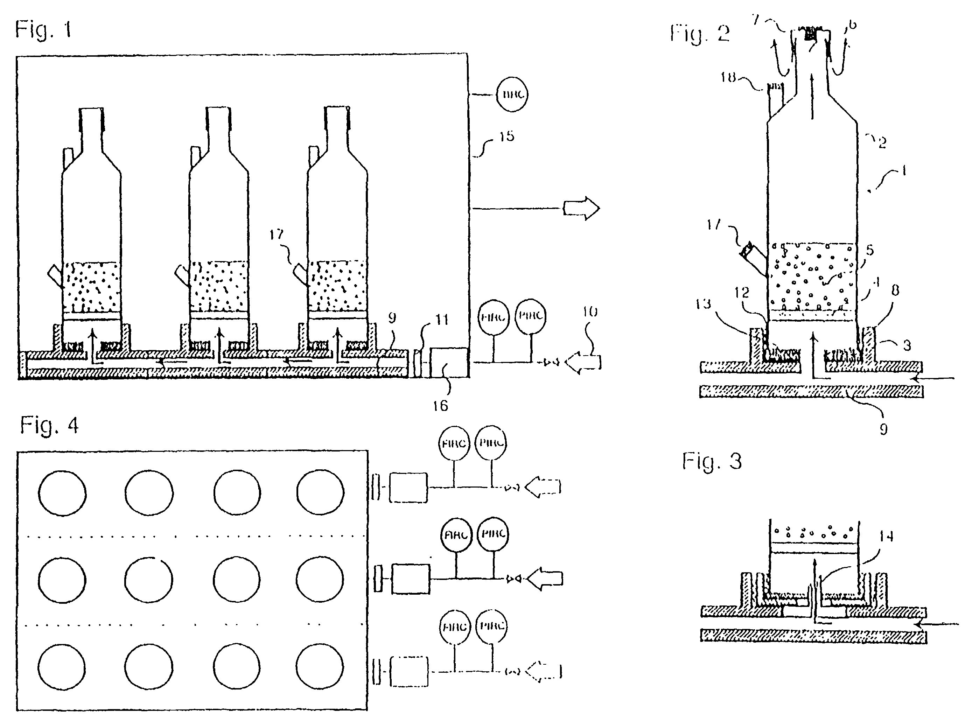

Fig. 1 zeigt eine Anordnung mit einer Mehrzahl von Kulturflaschen 1, die in Fig. 2 näher ausgeführt sind. Ein allgemein zylindrisches Glasgefäß 2, welches am Boden 3 offen oder mit einer Öffnung zur Einleitung von Gas versehen ist, enthält im unteren Teil eine poröse Filterplatte 4 (z.B. aus Borosilikatglas) insbesondere über die gesamte Querschnittsfläche, durch die das Gas in die darüberstehende Flüssigphase 5 (Medium mit Mikroorganismen) eingetragen wird. Am Kopf der Flasche 1 befindet sich eine Öffnung 6 zur Aufnahme von Standardvorrichtungen wie z. B. Sterilstopfen oder Aluminiumkappen 7, die ein Entweichen des Gases ohne Kontamination des Glasgefäßinhalts ermöglichen.

Mehrere Kulturflaschen 1, bevorzugt mit einem umschlossenen Volumen von 10 bis 1000 ml und einem Durchmesser von 10 bis 100 mm sind (insbesondere) gleichverteilt in napfartigen Aufnahmeplätzen 3 eines Gasverteilers 9 befestigt, der mit entsprechenden Anschlußstutzten zur Gasversorgung 10 der einzelnen Kulturflaschen 1 versehen ist. Dieser Gasverteiler 9 besitzt neben den Aufnahmeplätzen zur Gasversorgung und Befestigungsmitteln für die einzelnen Kulturflaschen eine oder mehrere zentrale Gaszufuhreinrichtungen, die jeweils mit einem Sterilfilter 11 versehen sind. Der Gasverteiler 9 ist zusammen mit den daran befestigten Kulturflaschen 1 autoklavierbar.

Die Fixierung der Kulturflaschen 1 in bzw. an den Aufnahmeplätzen 8 erfolgt zweckmäßigerweise über Verschraubungen oder Bajonetverschlüsse (bei 12) mit Dichtungsringen 13. In den Aufnahmeplätzen des Gasverteilers 9 sind zur Aufnahme der Kulturflaschen 1 Anstechvorrichtungen 14 angebracht, wie in Fig. 3 angedeutet, die eine Boden-Membran der Flasche durchstechen. Damit ist eine getrennte Sterilisation und nachträgliche Sterilverbindung von Gasverteiler und Kulturflaschen möglich.

Zur Temperierung ist der Gasverteiler mit den einzelnen Kulturflaschen in einem temperierten Gehäuse 15 installierbar, bzw. mit einer Abdeckhaube versehen, so daß die Reaktionstemperatur geregelt werden kann.

Die Medien und die Animpfbiomasse können nach dem Autoklavieren mit Standardtechniken steril zugegeben werden, um den Prozeß zu starten. Hierbei ist ein Flüssigvolumen 5 bis maximal 50 % des umschlossenen Volumens der Kulturflaschen besonders günstig, um ein Ausschäumen bei eventueller Schaumbildung zu verhindern.

Um auch im unbegasten Zustand ein Eindringen von Medium in den Gasverteiler zu unterbinden, sind die Kulturflaschen mit porösen Filterplatten ausgestattet, deren Porendurchmesser so fein ist, daß der hydrostatische Druck der darüberstehenden Flüssigkeitssäule nicht ausreicht, die Kapillaren der porösen Filterplatte zu füllen. So können im allgemeinen Filterplatten mit einem Porendurchmesser von maximal 20 µm Flüssigkeitssäulen von ca. 10 cm Höhe im unbegasten Zustand oberhalb der porösen Filterplatten halten. Zusätzlich kann die innere Oberfläche der porösen Filterplatten mit bekannten Mitteln hydrophobisiert werden. Besonders geeignete Techniken zur Herstellung von hydrophoben Glasoberflächen sind aus der Silan- bzw. Siliconchemie bekannt.

Das zur Begasung eingesetzte Gas wird vor dem Einleiten in den Sterilfilter 11 des Gasverteilers 9 mit Wasserdampf in einem Gasbefeuchter 16 (z.B. mittels entsprechender Waschflaschen) bei der eingestellten Reaktionstemperatur gesättigt, um eine Volumenabnahme in den begasten Säulen durch Verdunstung zu unterbinden.

Der Gasvolumenstrom (z.B. Luft oder mit Sauerstoff angereichertes Gasgemisch) kann auf Volumenströme z.B. bis zu 2,5 l Gas/l Reaktionsvolumen * Minute) vorgegeben werden. Bei Einsatz mehrerer parallel angeordneter Gaszufuhreinrichtungen für jeweils 1 Reihe von begasten Säulen kann für jede der unterschiedlichen Gaszufuhreinrichtungen ein anderer Gasvolumenstrom vorgegeben werden, wie in Fig. 4 angedeutet ist.

Zur Probenahme während der Reaktion sind die Kulturflaschen ggf. mit seitlichen Probenahmestutzten 17 und Septum versehen, über welches mit Hilfe einer Kanüle jederzeit eine definierte Probemenge steril entnommen werden kann.

Zur Aufnahme von Meßsonden (z.B. zum Messen von pH-Wert oder pO₂-Wert) ist ggf. am Kopf der begasten Säulen ein weiterer Stutzen 18 angebracht.

Bei Verwendung von Aluminiummkappen ('Alucaps') 7 zur kontaminationsfreien Entfernung des Gases aus den begasten Säulen ist ggf. durch Anbringen eines Septums und Anbindung von miniaturisierten Substratleitungen die kontinuierliche Zudosierung von Substraten oder Korrekturmittel während der Reaktion möglich.

Als Anwendungsgebiete sind
- die biotechnologische Forschung (als Alternative zur bisher üblichen Verwendung von Schüttelkolben) und
- die industrielle Prozeßentwicklung zur Herstellung von Produkten mit Mikroorganismen (mehr als 90 % der Experimente, die im Rahmen einer industriallen Prozeßentwicklung durchgeführt werden, erfolgen im Schüttelkolben) zu nennen.

Es folgen Beispiele, welche die Tauglichkeit der erfindungsgemäßen Anordnung veranschaulichen:

Beispiel 1: Vergleich der volumetrischen Sauerstoffeintragskoeffizienten (k_{L}a) der erfindungsgemäßen Kulturflaschen (begaste Säulen), herkömmlichen Schüttelkolben und Labor-Rührreaktor.

Der volumetrische Sauerstoffeintragskoeffizient k_{L}a wurde nach der Sulfit-Methode in Kulturflaschen (Durchmesser 60 mm) mit porösen Filterplatten aus Borosilikatglas (Porengröße 10 - 16 µm) bei einem Arbeitsvolumen von 150 ml und verschiedenen Gasdurchsätzen bestimmt (T = 30°C). Die erhaltenen Meßergebnisse (siehe Fig. 6) zeigen k_{L}a-Werte zwischen 0.05 und 0.15 s⁻¹. Zum Vergleich sind Daten aufgetragen, die unter vergleichbaren Bedingungen im Schüttelkolben und im Labor-Rührreaktor ermittelt wurden (Sulfit-Methode, T = 30°C).

Es ist deutlich zu erkennen, daß die volumetrischen Sauerstoffeintragskoeffizienten der Kulturflaschen (begaste Säulen) deutlich über den im Schüttelkolben erreichten Werten liegen und die Großenordnung des Labor-Rührreaktors erreichen.

Beispiel 2: Vergleich der Kultivierung von Corynebacterium glutamicum in erfindungsgemäßen Kulturflaschen (begaste Säulen) und in herkömmlichen Schüttelkolben.

Zum Vergleich des Fermentationsverlaufes in Kulturflaschen (begaste Säulen) und den herkömmlich im Labor verwendeten Schüttelkolben wurden Parallelansätze durchgeführt. Als Beispielsystem wurde die L-Isoleucin-Herstellung mit einem Corynebacterium glutamicum Stamm (KK47x15) des Instituts für Biotechnologie des Forschungszentrums Jülich gewählt.

Die porösen Filterplatten der im Beispiel 1 verwendeten Kulturflaschen (begaste Säulen) wurden vor dem Einsatz zur Fermentation wie folgt hydrophobisiert:
- Zur Vorbehandlung der Glasoberfläche wurden die begasten Säulen mit 5% Salpetersäure 4 h unter Rückfluß gekocht. Nach einer 12 h Reinigung mit destilliertem Wasser wurden bei 150°C getrocknet.
- Zur kovalenten Bindung von Methylgruppen an die Glasoberfläche der porösen Filterplatten werden die begasten Säulen mit 5% Trimethylchlorsilan in Chloroform 4 h unter Rückfluß gekocht. Anschließend wird 3 fach mit Chloroform gewaschen und 43 h bei 50°C im Wassertrahlvakuum getrocknet. Danach erfolgt eine 12 h Reinigung mit destilliertem Wasser mit anschließender Trocknung.

Herstellung der Vorkultur: 30 ml sterilisiertes Komplexmedium werden mit 20 ml steriler Glucoselösung (100 g/l) steril im 1000 ml Schüttelkolben vermischt und mit 2% Inocculum aus der Stammhaltung 12 h bei 30°C und 155 U/min inkubiert.

Nach der Hitzesterilisation der 1000 ml Schüttelkolben und der Kulturflaschen (begaste Säulen, Porendurchmesser der porösen Filterplatten: 10 - 15 µm) wurden jeweils 90 ml sterilisiertes Medlum und 10 ml Inocculum aus der Vorkultur steril zugegeben (Glucose 20 g/l). Die Schüttelkolben wurden im Schüttelinkubator bei 30°C und 155 U/min kultiviert, die Kulturflaschen (begaste Säulen) im Inkubator bei 30°C und 0.6 l/min befeuchteten Luft.

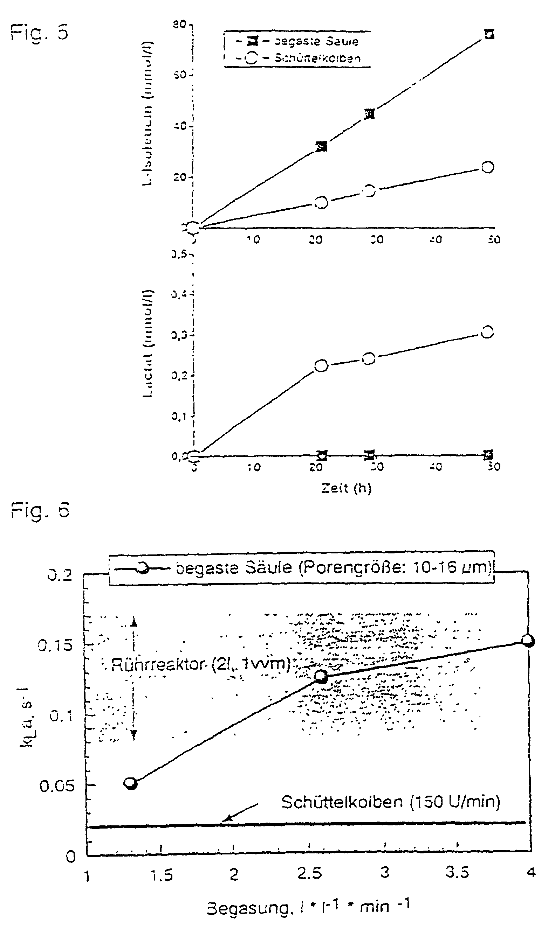

Die Meßergebnisse zeigen: Mit den Kulturflaschen (begaste Säulen) lassen sich aufgrund des weitaus besseren Sauerstoffeintrags 4fach höhere L-Isoleucinkonzentrationen erreichen (siehe Fig. 5). Dabei konnte über eine Lactatanalytik nachgewiesen werden, daß in den begasten Säulen keine Sauerstofflimitierung auftritt (kein Gärprodukt Lactat nachweisbar), während die Schüttelkolben sauerstofflimitiert waren (Lactat nachweisbar, siehe Fig. 5).

## Patentansprüche

1. Vorrichtung zur Serien-Kultivierung von Mikroorganismen bzw Zellen in begasten Flüssigkeitssäulen, bestehend aus Kulturflaschen (1), die oberhalb ihres Bodens bzw ihrer Bodenoffnung (3) mit einer gasdurchlässigen porösen Filterplatte (4) ausgestattet sind, deren Porenfeinheit und Hydrophobizität ausreichen, den Flüssigkeitsablauf aus einer darüber befindlichen Kulturflüssigkeitssäule zu unterbinden, und die uber ihren Boden bzw ihrer Bodenoffnung (3) mit einer Sterilgaszulertung, die Aufnahmeplatze (8) für die Kulturflaschen (1) aufweist, gasdicht verbunden bzw. zu verbinden sind,
**gekennzeichnet durch**
ggf auswechselbare und/oder als Schließventil ausgebildete Anstechvorrichtungen (14) im Aufnahemplatzboden (8) und Kulturflaschen (1) mit perforierbarem Membran-Boden.

2. Vorrichtung nach Anspruch 1,
**gekennzeichnet durch**
einen als Gasverteiler (9) wirkenden, mit Sterileinlaß für Gas versehenen gasdichten Doppelboden für die Sterilgazuleitung, dessen obere Platte mit einer Serie von Gasauslaßoffnungen aufweisenden Aufnahmeplatzen (8) für den abgedichteten Einsatz der Kulturflaschen (1) versehen ist.

3. Vorrichtung nach Anspruch 1 oder 2,
**gekennzeichnet durch**
saulenformige Kulturflaschen (1) mit einer oberen Verschlußkappe (7) mit Septum-Einsatz.

4. Vorrichtung nach einem der vorhergehenden Ansprüche,
**gekennzeichnet durch**
einen seitlichen Stutzen (17) mit Septum-Verschluß für Probenahmen.

5. Vorrichtung nach einem der vorhergehenden Ansprüche,
**gekennzeichnet durch**
Aufnahmeplatze (8), die napfartig mit Durchlaß zum Doppelbedenraum ausgebildet und mit am unteren Kulturflaschenumfang vorgesehenen zusammenwirkenden Verbindungselementen (12) versehen sind, die zur Herbeiführung eines ausreichenden Anpreßdrucks an einer Ringdichtung (13) zwischen unterem Flaschenende und Gasverteiler (9) geeignet sind.

6. Vorrichtung nach Anspruch 5,
**gekennzeichnet durch**
Schraub- oder Bajonetverbindungen als Verbindungselemente (12).

7. Vorrichtung nach einem der vorhergehenden Anspruche,
**gekennzeichnet durch**
eine die Flaschenserie übergreifende Abdeckhaube mit Thermostatisierung (15)

8. Vorrichtung nach einem der vorhergehenden Ansprüche,
**gekennzeichnet durch**
einen oder mehrere den Doppelboden in unterschiedliche Abteile mit jeweils eigener Gasversorgung unterteilende Trennwande.

9. Vorrichtung nach einem der vorhergehenden Ansprüche,
**gekennzeichnet durch**
jeweils mit Sterilfilter (11) und Gasbefeuchter (16) versehene Gasversorgungen.

10. Vorrichtung nach Anspruch 9,
**gekennzeichnet durch**
Einrichtungen, die die Gasbefeuchter (16) auf gleicher Temperatur halten wie den Kulturraum.

11. Vorrichtung nach einem der vorhergehenden Anspruche,
**gekennzeichnet durch**
Druck- und Durchtlußmesser in der Sterilgaszuleitung.

12. Verwendung einer Vorrichtung gemäß einem der Ansprüche 1 bis 11 zur Kultivierung von Mikroorgarnismen bzw Zellen.

## Claims

1. Device for series cultivation of micro-organisms or cells in gasified liquid columns, comprising culture bottles (1) which are above their bottom or their bottom opening (3) fitted with a gas-permeable porous filter plate (4) the degree of pore fineness and hydrophobicity of which is sufficient to check the liquid discharge of a culture liquid column located thereabove and which are in a gastight manner joined or to be joined via their base or their base opening (3) to a sterile gas feed having accommodation spaces (8) for the culture bottles (1), **characterised by** piercing devices (14) in the accommodation area bottom (8), which are if appropriate exchangeable and/or designed as a closure valve, and culture bottles (1) with perforatable membrane bottom.

2. Device according to Claim 1, **characterised by** a gastight double bottom for the sterile gas feed, which acts as a gas distributor (9) and which is provided with a sterile inlet for gas, the upper plate of which is provided with accommodation places (8) the sealed use of the culture bottles (1) with a series of gas outlet openings.

3. Device according to Claim 1 or 2, **characterised by** columnar culture bottles (1 ) with an upper sealing cap (7) with septrum insert.

4. Device according to one of the above claims, **characterised by** a lateral socket (17) with septrum closure for sampling.

5. Device according to one of the above claims, **characterised by** accommodation places (8) which are designed like a bowl with a passage to the double-bottom area and provided with co-acting connecting elements (12) at the lower culture bottle circumference which are suitable for producing a sufficient press-on pressure on an annular seal (13) between lower bottle end and gas distributor (9).

6. Device according to Claim 5, **characterised by** screwing or bayonet connections as connecting elements (12).

7. Device according to one of the above claims, **characterised by** a covering hood with thermostatisation (15) which reaches over the bottle series.

8. Device according to one of the above claims, **characterised by** one or more separating walls which divide the double bottom into different sections with respective own gas supply.

9. Device according to one of the above claims, **characterised by** respective gas supplies provided with sterile filter (11) and gas humidifier (16).

10. Device according to Claim 9, **characterised by** devices which keep the gas humidifiers (16) at the same temperature as the culture area.

11. Device according to one of the above claims, **characterised by** pressure and throughflow meters in the sterile gas feed.

12. Use of a device according to one of Claims 1 to 11 for cultivation of micro-organisms or cells.

## Revendications

1. Dispositif pour la culture en série de micro-organismes ou de cellules dans des colonnes de liquide gazéifié, constitué de flacons de culture (1) qui sont munis au-dessus de leur fond ou de leur ouverture du fond (3) d'une plaque filtrante (4) poreuse perméable aux gaz, dont la finesse des pores et l'hydrophobicité est suffisante pour interdire l'écoulement des liquides d'une colonne de liquide de culture se trouvant dessus, et qui sont reliés ou doivent être reliés de manière étanche au gaz, par le biais de leur fond ou de leur ouverture sur le fond (3), par une conduite d'amenée de gaz stérile, présentant des sites d'accueil (8) pour les flacons de culture (1), **caractérisé par** éventuellement des dispositifs de percée (14) remplaçables et/ou sous forme de clapet de fermeture dans le fond des sites d'accueil (8) et par des flacons de culture (1) dotés d'un fond membranaire perforable.

2. Dispositif selon la revendication 1, **caractérisé par** un double fond pour la conduite d'amenée des gaz stérile, étanche au gaz et doté d'une admission stérile pour les gaz, agissant comme distributeur de gaz (9), et dont la plaque supérieure est munie d'une série de sites d'accueil (8) présentant des ouvertures d'échappement des gaz pour la mise en oeuvre étanche des flacons de culture (1).

3. Dispositif selon la revendication 1 ou 2, **caractérisé par** des flacons de culture (1) en forme de colonne, dotés d'un capuchon (7) supérieur avec mise en oeuvre septique.

4. Dispositif selon l'une des revendications précédentes, **caractérisé par** un ajutoir latéral (17) muni d'une fermeture septique pour les prélèvements d'échantillons.

5. Dispositif selon l'une des revendications précédentes, **caractérisé par** des sites d'accueil (8) conçus à la manière de godets avec un passage vers une chambre de double fond, et munis d'éléments de liaison (12) coopérants, prévus sur le contour inférieur des flacons de culture, lesdits éléments étant appropriés à la génération d'une pression d'appui suffisante sur un joint circulaire (13) situé entre l'extrémité inférieure du flacon et le distributeur de gaz (9).

6. Dispositif selon la revendication 5, **caractérisé par** des jonctions à vis ou à baïonnette à titre d'éléments de jonction (12).

7. Dispositif selon l'une des revendications précédentes, **caractérisé par** un couvercle englobant la série de flacon avec thermostatisation (15).

8. Dispositif selon l'une des revendications précédentes, **caractérisé par** une ou plusieurs parois de séparation divisant le double fond en différents compartiments avec à chaque fois leur propre alimentation en gaz.

9. Dispositif selon l'une des revendications précédentes, **caractérisé par** des alimentations en gaz dotées respectivement d'un filtre stérile (11) et d'un humidificateur de gaz (16).

10. Dispositif selon la revendication 9, **caractérisé par** des appareils qui maintiennent les humidificateurs de gaz (16) à la même température que la chambre de culture.

11. Dispositif selon l'une des revendications précédentes, **caractérisé par** un manomètre et un débitmètre dans la conduite d'amenée de gaz stérile.

12. Utilisation d'un dispositif selon l'une des revendications 1 à 11 pour cultiver des micro-organismes ou des cellules.
